Europäisches Patentamt

European Patent Office

Office européen des brevets

(19)

(11) Publication number: **0 267 724 B1**

## EUROPEAN PATENT SPECIFICATION

(12)

(45) Date of publication of patent specification: **04.03.92** (51) Int. Cl.5: **G01N 33/52**, C09D 11/02

(21) Application number: **87309691.1**

(22) Date of filing: **02.11.87**

(54) **Test devices with reagent layers formed by printing processes.**

(30) Priority: **31.10.86 GB 8626081**

(43) Date of publication of application:
**18.05.88 Bulletin 88/20**

(45) Publication of the grant of the patent:
**04.03.92 Bulletin 92/10**

(84) Designated Contracting States:
**AT BE CH DE ES FR GB GR IT LI NL SE**

(56) References cited:
EP-A- 0 016 387        EP-A- 0 115 873
EP-A- 0 170 375        EP-A- 0 171 148
EP-A- 0 186 286        EP-A- 0 200 539
US-A- 3 993 451        US-A- 4 216 245

(73) Proprietor: **UNILEVER PLC**
**Unilever House Blackfriars P.O. Box 68**
**London EC4P 4BO(GB)**

(84) Designated Contracting States:
**GB**

(73) Proprietor: **UNILEVER NV**
**Burgemeester s'Jacobplein 1 P.O. Box 760**
**NL-3000 DK Rotterdam(NL)**

(84) Designated Contracting States:
**BE CH DE ES FR GR IT LI NL SE AT**

(72) Inventor: **Baginski, Edward**
**11 Castle Mews**
**Wellingborough NN8 1NX(GB)**
Inventor: **Cattell, Alan Frank**
**4 Brittons Close**
**Sharnbrook, Bedford MK44 1PN(GB)**

(74) Representative: **Butler, David John et al**
**Unilever PLC Patents Division P.O. Box 68**
**Unilever House**
**London EC4P 4BO(GB)**

Rank Xerox (UK) Business Services

## Description

This invention relates materials for microchemical testing, and to their preparation and use, and especially to test devices containing reagent layers which have been formed by printing processes. Thus the invention relates especially for example to processes for printing materials which are to form functional or reactive portions of devices such as carriers of chemical reagents or electrical and electrochemical devices and components. The invention also relates to certain printable compositions which can be used in such processes and the devices produced by the processes.

Screen-printing and contact printing are well known in themselves, as are many compositions of ink and coatings to be applied by their use. It is also common for printing inks to contain particulate material, e.g. as in EP 0 202 656 (Canon KK).

EP 0 171 148 and 0 170 375 (Unilever) describe the construction of capillary-fill devices, including the deposition of adhesive patches containing solid particulate or fibrous material to regulate the spacing apart of opposite walls of the devices.

EP 0 188 232 (Nippon Paint KK) discloses radiation- curable liquid resin compositions incorporating 0.01-6 $\mu$m polymer microparticles, and also mentions a use of such materials for immobilising enzymes.

GB 2 040 946 (STC) discloses inks for screen printing electrical components, based on phenol resins, and loaded with silver flake or powdered silica.

Also included in the prior art are a number of enzyme electrode measurement methods, in one of which an enzyme (urease) is chemically bound to a ptfe membrane which is an integral part of an ammonia gas membrane electrode incorporating an ammonia permeable membrane, (e.g. M Mascini and C G Guilbault, Anal Chem. 49 (6) 1977, pp 795-798).

Also known are electrochemical cells mounted within capillary-fill devices, as described in European Patent Application No. 0 170 135 (Unilever).

We have encountered difficulties in the formulation of materials which are to be printed in a pattern-wise manner and which are to have chemical reactivity or defined chemical or electrochemical properties. This invention aims to solve certain such difficulties.

Thus, an aim of the invention is to provide conveniently printable compositions for the placing of small active areas of layers of desired reagents in chemical test devices, e.g. for immunological and/or electrochemical testing. The details of such tests in themselves are not however the subject of the present invention, and reference is made to the prior art for such details.

The present invention also provides useful processes for depositing reactive materials on to or to form such electrodes, or on to or to form composite test devices embodying electrodes, and relates also to corresponding compositions, electrodes and devices so formed.

According to one aspect of the present invention there is provided a test device for carrying out a microchemical test on a reaction liquid, comprising:

a) a container for receiving the reaction liquid, the container having a surface which contacts said reaction liquid in use; and

b) material, including a chemically or electrochemically active component, deposited in a defined pattern on said surface of the container, the material being present in an amount in the range 1 to 40 mg/sq cm and having been deposited on said surface by printing a composition comprising solid particles having a particle size in the range 0.5 to 50 $\mu$m together with a polymeric binder.

In another aspect the invention also provides a process for the manufacture of a test device in accordance with the invention, comprising:

a) providing a printable composition including a chemically or electrochemically active component and comprising solid particles having a particle size in the range 0.5 to 50 $\mu$m together with a polymeric binder;

b) printing said printable composition to form a defined pattern of material on a surface of a substrate which is to form an inner surface of a container for receiving a reaction liquid, said material being present in an amount in the range 1 to 40 mg/sq cm; and

c) causing or permitting said printed material to dry or solidify.

The invention also includes within its scope a printable composition for printing a defined pattern of material on a surface substrate which is to form an inner surface of a container for a test device in accordance with the invention, comprising:

a) a chemically or electrochemically active substance;

b) solid particles having a particle size in the range 0.5 to 50 $\mu$m and

c) a polymeric binder.

The particles can compromise inert particles, e.g. ptfe or silica gel, and can be dispersed in a matrix comprising said active material and a polymeric binder, e.g. a water-soluble cellulose derivative. Alternatively, said active material can be used in solid finely-divided form, and dispersed in such a polymeric binder.

The chemically or electrochemically active material can be water-soluble, e.g. selected from enzymes, antigens, antibodies, electron transfer agents able to react with enzymes, or pH buffer materials.

Alternatively, the active material may be water-insoluble, e.g. an ion-selective ionophore material.

Particle size can usefully be in the range from at least about 3 $\mu$m to at least about 15 $\mu$m and the particles can be selected from ptfe, silica-gel, polystyrene, polymer latex, and comminuted solids comprising the chemically or electrochemically active material to be deposited. The particles can be used in an amount from at least about 1 percent to at least about 60 percent, e.g. about 5 to about 15 percent, by weight of the printable composition.

The invention also provides a printable composition for printing a layer of a chemically or electrochemically active material on to a surface of a substrate to form a device as described herein, comprising (a) a chemically or elctrochemically active substance to be deposited, dissolved in (b) a (e.g. aqueous) solvent for said substance (a), (c) a polymeric binder or thickener dissolved or dispersed in solvent (b), (c) from 1 to 15 percent by weight of a dispersed finely-divided inert particulate material with a particle size in the range 1 to 50 $\mu$m said composition having a viscosity in the range 0.1 to 25.0 Pa.s (1 to 250 poise) at 25 deg.C.

An alternative form of printable composition comprises (a) a polymeric binder or thickener dissolved or dispersed in a (e.g. nonaqueous) solvent, having dispersed therein (b) from 5 to 60 percent by weight of a chemically or electrochemically active (e.g. water-soluble or water-dispersible) substance to be deposited, in finely-divided solid particulate form having a particle size less than 20 micron, said composition having a viscosity in the range 0.1 to 25.0 Pa.s (1 to 250 poise), preferably at least about 15.0 Pa.s (150 poise), at 25 deg.C.

The printable composition can usefully show shear-thinning of at least about 0.1 log viscosity per tenfold increase of shear rate in the range of shear rates about 10 to 100 reciprocal seconds, and has a viscosity at 200 reciprocal seconds at 25 deg.C. in the range 0.1 to 3.5 Pa.s (1 to 35 poise).

The preferred printing techniques for use in this invention are screen-printing and variants thereof including contact printing e.g. using stencils. Layers of printable composition can be laid down at thicknesses ranging up to about 400 $\mu$m and give dried layers at up to about 40mg/sq cm, but it is often preferred to work at about a tenth of these levels, e.g. in the range of at least about 1 to at least about 10 mg/sq cm.

The particles can for example be about 0.05 $\mu$m to 25 $\mu$m in size, e.g. above about 0.5 $\mu$m, especially about 3 to 15 $\mu$m. We have found that the incorporation of such particles can usefully increase viscosity at low shear, and/or impart or enhance shear-thinning properties in the materials to be printed, and/or reduce extensional viscosity of the materials to be printed.

Corresponding advantages can be achieved in accordance with examples of certain aspects of the invention by incorporating hydrophilic particles into hydrophilic flowable materials, and hydrophobic particles into hydrophobic flowable materials.

For example, ptfe particles (from BDH Laboratory Chemicals) of about 5 $\mu$m in size can be used, as can silica gel particles (same source) about 15 micron in size. The particles can for example be roughly spherical, or they can be porous in themselves. Also usable are for example polystyrene and other polymer latices, either chemically modified or unmodified, preferably uncharged, but possibly charged.

Such particles can usefully be incorporated in:

(i) Materials to be printed to form membranes, e.g. electroactive membranes, such as chemically selective membranes, e.g. ion-selective membranes to overcoat electrochemical electrodes. Suitable compositions for such membranes include for example compositions based on pvc including ionophore, plasticiser, and optionally solvent.

(ii) Materials to be printed to form reagent layers, releasable or otherwise, e.g. compositions such as aqueous compositions including salts, oxidants, reductants, enzymes, and/or enzyme substrates, optionally including thickeners, (e.g. polyvinyl alcohol or polyvinylpyrrolidone) and/or humectants (e.g. saccharides such as glucose, sucrose, lactose or maltose).

(iii) Materials to be printed to form structural components of printed devices, e.g. glue tracks or spacer tracks, partitions or barriers.

The particles may include conductive particles, e.g. in connection with materials to form ion-selective membranes.

The invention includes for example applying such materials on printed layers in succession, optionally allowing for intermediate drying and/or curing: e.g. the formation of overlayers, such as for example porous membranes such as cellulose acetate membranes overlying a layer of another material such as an ion-selective membrane or a water-soluble layer capable of releasing its content into contacting aqueous liquid. Such overlayers may be useful to control diffusion or access or exit of materials to or from the underlying layer or device component.

The quantity of particles can be chosen and adjusted to suit the viscosity requirements. For example, 20-40% v/v of ptfe particles 3-15 $\mu$m can be used in pvc membrane printing compositions. Lower amounts may be needed of smaller particles, and higher amounts of larger particles. The particles will often be sufficiently dilute in the printing compositions that they are out of direct contact with each other in the resulting dried and/or cured layers, e.g. out of electrical contact where conductive particles are used.

The surfaces to be printed may be flat or curved. The printing process may be a screen-printing process involving a mesh and squeegee, or for example a contact (or offset) roller-printing process in which there may be used an apertured mask, or any other suitable printing process for depositing a pattern of detail upon a substrate. Particle size is related to size of mesh (where used) so that the particles easily pass through - e.g. particle size may be less than half the mesh aperture size.

The printing compositions are preferably degassed in normal and known manner before use, and if necessary and appropriate the particles disaggregated before use, e.g. by stirring.

A suitable initial viscosity (i.e. at low shear rates) for the compositions is for example in the range of 0.1 to 25.0 Pa.s. (1 to about 250 poise), e.g. at least about 15.0 Pa.s (150 poise), and preferably of the order of about 20.0 Pa.s (20,000 cps, i.e. 200 poise), when measured in a Brookfield RVT viscometer, spindle 6, at 20 rpm, and 25 deg.C.

It is common to find that the viscosity of binders in usual nonaqueous systems is higher than in the aqueous case. It is useful to try and use as high a proportion of solids as possible in the (usually) nonaqueous compositions in which the reagents form all or part of the particulate materials, as in that case the particulates comprise the active substances. In the (usually) aqueous systems comprising inert particles we try and use as little as possible of the particles which are only inert materials. 10 - 15% w/w or in some cases as low as about 1% has often been found an adequate particle content for the latter systems, while particle contents of up to about 60% can be usable in the former systems.

Preferably the compositions also show appreciable shear-thinning to viscosity in the range of about 0.1 to 3.5 Pa.s. (1 to 35 poise) at a shear rate of 100 reciprocal seconds, preferably at least about 2.0 Pa.s (20 poise), e.g. about 3.0 Pa.s (30 poise).

In practice it has been found that many suitable examples of the compositions show a shear-thinning at a rate corresponding to a reduction of at least about 0.1 in log viscosity (base 10) per tenfold increase in shear rate within a range of shear rates from about 10 to about 100 reciprocal seconds, and up to 0.6 log viscosity units, preferably in the range 0.3 to 0.6 log viscosity though in certain cases it may not be practicable to formulate for shear-thinning rates as much as these.

In practice, it has been found that many of the screen-printing compositions according to the invention are of suitable properties for printing when the directions given herein are followed and they have had a consistency like that of mustard or a mayonnaise sauce.

It is sometimes found that uniformity of the dried printed layers can be improved by including a small amount of surfactant in the printing composition, e.g. of the order of about 0.1%, e.g. a neutral Tween (trade mark) surfactant.

Illustrative embodiments of this invention are given in and by the following examples.

Example 1

One form of printing composition can be made by the following steps:

(a) dissolve the reagent(s) to be deposited in a chosen solvent, e.g. water or other aqueous solvent such as a buffer suitable for a biochemical reagent to be handled;

(b) add a polymer binder, such as for example polyvinylpyrrolidone (pvp), polyvinylalcohol (pva), and/or sodium carboxymethylcellulose (scmc);

(c) add fine inert particle material, e.g. in an amount in the range of about 1 to 15% by weight of the final composition.

In each case the ingredients should be thoroughly mixed, e.g. in a ball mill or cavitation mixer.

Preferred conditions for making compositions of this form are as follows:

A preferred polymer binder is a mixture of pvp 30% and pva 5% (both on w/v basis as a proportion of the liquid part of the final composition).

A preferred type of particles for thickening the composition is silica gel particles of average particle size about 15 $\mu$m, 5 to 15%, e.g. 10% by weight of the final composition. An alternative example of particles for thickening purposes is Aerosil (registered Trade Mark) 200 from Degussa, average particle size 1/4 $\mu$m chain-forming particles, which can be used in quantity such as 1 to 3% by weight.

Example 2

A second form of printing composition can be made as follows:

(a) take the reagent(s) to be deposited in solid form and comminute them, e.g. by grinding in a ball mill;

(b) disperse the comminuted reagent particles in a polymer solution.

A preferred example formulation of a printing composition of this kind, suitable for printing a reagent mixture essentially comprising glucose oxidase enzyme and potassium ferricyanide for the electrochemical measurement of glucose concentrations is as follows:

Grind a mixture of solids consisting of 64% potassium ferricyanide, 32% phosphate buffer salt for pH about 7, and 4% glucose oxidase enzyme, in a ball mill until nearly all the particles, are no more than 10 microns in size. It is often then found that the resulting mixture of particles are to the extent of about 90% within the size range 1 to 10 $\mu$m.

To the ground solids (50 parts by weight) is added first 40 parts by weight of a mixture of cellulose acetate (thickener/binder) in cyclohexanone (solvent), and then 10 parts by weight of isophorone (slow evaporating solvent, obtainable from Aldrich Chemical Co.). In each case the ingredients should be thoroughly mixed, e.g. with a ball mill or cavitation mixer.

## Example 3

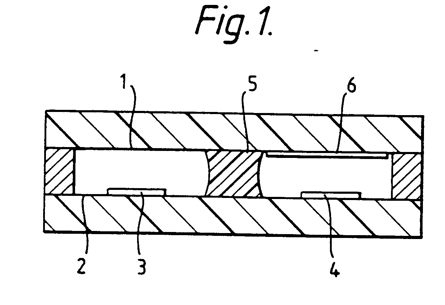

Figure 1 of the accompanying drawings shows in diagrammatic cross-sectional scheme an example of an electrode-containing capillary cell device to illustrate one example of the environment within which reagent layer(s) can be deposited according to an embodiment of the invention. (See also EP 0 225 061.) In Figure 1, at numerals 1 and 2, there are shown opposite walls of the capillary cell. Other structure is omitted from the diagrammatic drawing apart from electrode(s) and reagent layer(s) and a partition which for certain uses will be omitted. Such other structure can include e.g. further circumferential sealing materials, handling pieces, sample intake lip, and intake aperture(s). The capillary gap can conveniently be of the order of 0.1-1 mm.

The capillary cell device is of a size to be handled easily, e.g. about 5cm × 2.5cm. Alternative examples can be smaller, e.g. 2cm × 1cm. The device comprises an upper (e.g. plastics, glass, or ceramic) plate and a lower (e.g. similar) plate (about 1mm thick) fixed together in parallel opposed and spaced relation, less than 1mm apart, by bonding tracks of suitable (e.g. epoxy) adhesive to form a capillary cell cavity, open at one or both ends, which communicates with the outside through a first discontinuity in the bonding arranged to form a cell aperture at one side of the plates. Another discontinuity can be present at the other end of the bonding tracks, to leave another aperture, to allow exit or air when a sample liquid is loaded into the cell. One plate is larger than the other plate and has an end portion extending away from the aperture. This end portion acts as a platform or threshold or lip onto which a droplet of sample liquid can be applied, so that this liquid can be made to fill the capillary cell cavities by capillary flow. The cavity attracts and contains a definite and adequately reproducible volume of liquid when loaded in this way.

Spaced-apart electrodes 3 and 4 are shown as layers fixed to the surface of wall 2 of the cell. Electrodes 3 and 4 can be made according to the intended use of the device: in the present embodiment they are silver-silver halide electrodes (for potentiometry), most preferably silver-silver chloride electrodes, made by applying and drying conductive paint onto the surface of wall 2, the conductive paint being a silver-particle-containing conductive paint as commercially available and used in the fabrication of hybrid electronic circuitry. In alternative embodiments for amperometric use the electrodes can usefully be for example noble-metal electrodes e.g. silver or gold, or carbon electrodes.

In the device shown in Figure 1 a membrane barrier 5 less than 1 mm thick separates the two silver/silver chloride electrodes: this can be applied as a track of material comprising a mixture of pvc, solvent, plasticiser and ionophore (e.g., valinomycin in the case of a potassium-selective membrane) to one of the plates before the second plate is applied to form the capillary cell. The track of material can be applied for example by screen- printing, as can the other layers of materials to be deposited. A suitable example composition for the track of material is 1% valinomycin, 66% dioctyl sebacate and 33% polyvinylchloride dissolved i n tetrahydrofuran, e.g. at a concentration of about 0.08-0.1 gram of the mixture per ml of the solvent. Particles can be added to improve the printing qualities of the composition, e.g. inert particles as detailed below.

A releasable layer 6 containing potassium nitrate is coated on the wall of the capillary cell opposite one electrode. This coating is a releasable coating (e.g. a sucrose glaze) to be released into one compartment only and to dissolve in the sample liquid to give a standard concentration of potassium ion when this is taken up into the capillary cell.

In a preferred alternative, in place of sucrose, polyvinylpyrrolidone, (50% w/v) is used as a carrier base in which to dissolve the salt which is to form a releasable layer. Also, 2% cellulose acetate dissolved in cyclohexanone, is preferably sprayed lightly over the releasable coating to form when dry an additional thin surface layer which acts to retard the release of salt when the cell is filled. The salt/pvp layer dries quickly in atmospheric air after screen-printing, and the cellulose acetate is applied after it has dried.

A presently preferred example of materials to prepare an electrochemical analysis cell, according to one embodiment of the invention, is as follows:

A capillary-fill cell is made according to the general form shown in EP 0 225 061, with a preferred composition as follows to be used for the track of material to be screen-printed onto one of its internal surfaces to provide an electroactive barrier:

(a) In the case of a potassium-sensitive cell a screen-printing composition is preferably made as follows:

Mix 44.5g dioctyl phthalate, 5.5g pvc of m.w. 100,000, 12.5g cyclohexanone, together, and heat to dissolve the pvc, to give a solution. To this is added a solution containing : 0.5g valinomycin (in the case of a potassium-sensitive electrode) dissolved in 2.5 ml of tetrahydrofuran, (containing 5 mgm of potassium tetrakis-parachlorophenyl borate) at a temperature below 50 deg C, with stirring. To the mixture is added: 12.5g of 5-$\mu$m ptfe particles (from BDH) (slowly while stirring vigorously).

This composition can be used for screen-printing an ion-sensitive membrane.

(b) In the case of a sodium-sensitive cell, the composition is preferably varied as follows: Monensin (ionophore for making sodium-sensitive membranes) is used instead of valinomycin in a 1-gram amount, and with proportionately more THF to produce a solution which is then used in place of the valinomycin solution.

Among the practicable variations is the use of other kinds of small particles (e.g. about 20% silica gel particles by weight based on the weight of the whole composition, 15 $\mu$m in size as obtained from BDH Laboratory Chemicals), optionally together with a thickener such as pvp, as part of the aqueous-based compositions to be used for printing patchwise releasable reagent layers and hydrophilic (e.g. cellulose acetate) membrane layers.

## Example 4

(In this and the following examples, phosphate buffer means a substantially equimolar mixture of potassium hydrogen phosphate and dipotassium hydrogen phosphate, adjusted to give a pH of 7 when at approximately 1M concentration.)

A printing composition for screen-printing a layer of reagent material for a cell to measure glucose and sucrose electrochemically (amperometry etc of ferrocyanide produced by action of glucose oxidase on glucose, possibly liberated by invertase) is as follows:

Solids: 25.8% potassium ferricyanide, 13.3% phosphate buffer, 1.7% glucose oxidase, 0.8% invertase, and liquids: 41.7% of a solution of ethylcellulose (5%) in cyclohexanone, and 16.7% of a solution of cellulose acetate (10%) in cyclohexanone.

The solids were comminuted together in a ball mill as described elsewhere herein, then suspended in the liquids and screen-printed to give a density of about 3 mg/sq cm of the dried layer material.

The composition of the final coating after drying is:

Potassium ferricyanide - 57.7% phosphate buffer - 28.8% glucose oxidase - 3.6% invertase - 1.8% ethylcellulose - 4.5% cellulose acetate - 3.6%

## Example 5

A printing composition for screen-printing a layer of reagent material for a cell to measure glucose electrochemically is as follows:

Solids: 32.5% potassium ferricyanide, 16.5% phosphate buffer, 1% glucose oxidase, and liquid: 50% of a solution of cellulose acetate (10%) in cyclohexanone.

The solids were comminuted together in a ball mill as described elsewhere herein, then suspended in the liquid and screen-printed to give a density of about 3 mg/sq cm of the dried layer material.

The composition of the final coating after drying is:

Potassium ferricyanide - 59.1% phosphate buffer - 30% glucose oxidase - 1.8% cellulose acetate - 9.1%.

## Example 6

A printing composition for screen-printing a layer of reagent material for a cell to include the function of a quinhydrone pH electrode is as follows:

Solids: 58.8% sodium chloride, quinhydrone 2%, and liquid: 39.2% of a solution of cellulose acetate (10%) in cyclohexanone.

The solids were comminuted together in a ball mill as described elsewhere herein, then suspended in the liquid and screen-printed to give a density of about 3 mg/sq cm of the dried layer material.

The composition of the final coating after drying is:

sodium chloride - 91%; quinhydrone - 3%; cellulose acetate - 6%.

Variants of the present invention can be applied to facilitate the construction of devices such as those shown in for example EP 0 171 148, 0 170 375, 0 186 286, and 0 225 061.

## Claims

1. A test device for carrying out a microchemical test on a reaction liquid, comprising:

a) a container for receiving the reaction liquid, the container having a surface which contacts said reaction liquid in use; and
b) material, including a chemically or electrochemically active component, deposited in a defined pattern on said surface of the container, the material being present in an amount in the range 1 to 40 mg/sq cm and having been deposited on said surface by printing a composition comprising solid particles having a particle size in the range 0.5 to 50 $\mu$m together with a polymeric binder.

2. A test device according to claim 1, wherein said particles comprise inert particles, and are dispersed in a matrix comprising the active component and the polymeric binder.

3. A test device according to claim 2, wherein said inert particles comprise ptfe or silica gel.

4. A test device according to claim 2 or 3, wherein said polymeric binder comprises a water-soluble cellulose derivative.

5. A test device according to any one of the preceding claims, wherein said particles comprise the active component in solid finely-divided form, and are dispersed in the polymeric binder.

6. A test device according to claim 5, wherein the polymeric binder comprises a water-soluble cellulose derivative.

7. A test device according to any one of the preceding claims, wherein the particles have a particle size in the range 1 to 20 $\mu$m.

8. A test device according to claim 7, wherein the particles have a particle size i the range 3 to 15 $\mu$m.

9. A test device according to any one of the preceding claims, wherein the particles constitute between 1 and 60% by weight of the printable composition.

10. A test device according to claim 9, wherein the particles constitute between 5 and 15% by weight of the printable composition.

11. A test device according to any one of the preceding claims, wherein the material is present in an amount in the range 1 to 10 mg/sq cm.

12. A test device according to any one of the preceding claims, wherein the printable composition has a viscosity in the range 0.1 to 25.0 Pa.s (1 to 250 poise) at 25°C.

13. A test device according to claim 12, wherein the printable composition has a viscosity of at least about 15.0 Pa.s (150 poise) at 25°C.

14. A test device according to any one of the preceding claims, wherein the printable composition shows shear-thinning of at least about 0.1 log viscosity per tenfold increase of shear rate in the range of shear rates about 10 to 100 reciprocal seconds and has a viscosity at 200 reciprocal seconds in the range 0.1 to 3.5 Pa.s (1 to 35 poise) at 25°C.

15. A test device according to any one of the preceding claims, wherein said chemically or electrochemically active component is water-soluble and is selected from enzymes, antigens, antibodies, electron transfer agents able to react with enzymes, and pH buffer materials.

16. A test device according to any one of claims 1 to 14, wherein said chemically or electrochemically active material is an ion-selective ionophore material.

17. A process for the manufacture of a test device according to claim 1, comprising:

a) providing a printable composition including a chemically or electrochemically active component and comprising solid particles having a particle size in the range 0.5 to 50 $\mu$m together with a polymeric binder;
b) printing said printable composition to form a defined pattern of material on a surface of a substrate which is to form an inner surface of a container for receiving a reaction liquid, said material being present in an amount in the range 1 to 40 mg/sq cm; and
c) causing or permitting said printed material to dry or solidify.

18. A process according to claim 17, wherein said particles comprise inert particles, and are dispersed in a matrix comprising the active component and the polymeric binder.

19. A process according to claim 18, wherein said inert particles comprise ptfe or silica gel.

20. A process according to claim 18 or 19, wherein said polymeric binder comprises a water-soluble cellulose derivative.

21. A process according to claim 17, wherein said particles comprise the active component in solid finely-divided form, and are dispersed in the polymeric binder.

22. A process according to any one of claims 17 to 21, wherein the polymeric binder comprises a water-soluble cellulose derivative.

23. A process according to any one of claims 17 to 22, wherein the particles have a particle size in the range 1 to 20 $\mu$m.

24. A process according to claim 23, wherein the particles have a particle size in the range 3 to 15 $\mu$m.

25. A process according to any one of claims 17 to 24, wherein the particles constitute between 1 and 60% by weight of the printable composition.

26. A process according to any one of clais 17 to 24, wherein the particles constitute between 5 and 15% by weight of the printable composition.

27. A process according to any one of claims 17 to 26, wherein the material is present in an amount in the range 1 to 10 mg/sq cm.

28. A process according to any one of claims 17 to 27, wherein the printable composition has a viscosity in the range 0.1 to 25.0 Pa.s (1 to 250 poise) at 25°C.

29. A process according to claim 28, wherein the printable composition has a viscosity of at least about 15.0 Pa.s (150 poise) at 25°C.

30. A process according to any one of claims 17 to 29, wherein the printable composition shows shear-thinning of at least about 0.1 log viscosity per tenfold increase of shear rate in the range of shear rates about 10 to 100 reciprocal seconds and has a viscosity at 200 reciprocal seconds in the range 0.1 to 3.5 Pa.s (1 to 35 poise) at 25°C.

31. A process according to any one of claims 17 to 30, wherein said chemically or electrochemically active component is water-soluble and is selected from enzymes, antigens, antibodies, electron transfer agents able to react with enzymes, and pH buffer materials.

32. A process according to one of claims 17 to 30, wherein said chemically or electrochemically active material is an ion-selective ionophore material.

33. A printable composition for printing a defined pattern of material on a surface of a substrate which is to form an inner surface of a container for a test device according to claim I, comprising:

    a) a chemically or electrochemically active substance;
    b) solid particles having a particle size in the range 0.5 to 50 $\mu$m; and
    c) a polymeric binder.

34. A printable composition according to claim 33, wherein said particles comprise inert particles, and are dispersed in a matrix comprising the active component and the polymeric binder.

35. A printable composition according to claim 34 wherein said inert particles comprise ptfe or silica gel.

36. A printable composition according to claim 33, wherein said particles comprise the active component in solid finely-divided form, and are dispersed in the polymeric binder.

37. A printable composition according to any one of claims 33 to 36, wherein the polymeric binder comprises a water-soluble cellulose derivative.

38. A printable composition according to any one of claims 33 to 37, wherein the particles have a particle size in the range 1 to 20 $\mu$m.

39. A printable composition according to claim 38, wherein the particles have a particle size in the range 3 to 15 $\mu$m.

40. A printable composition according to any one of claims 33 to 39, where the particles constitute between 1 and 60% by weight of the printable composition.

41. A printable composition according to claim 40, wherein the particles constitute between 5 and 15% by weight of the printable composition.

42. A printable composition according to any one of claims 33 to 41, wherein the printable composition has a viscosity in the range 0.1 to 25.0 Pa.s (1 to 250 poise) at 25°C.

43. A printable composition according to claim 42, wherein the printable composition has a viscosity of at least about 15.0 Pa.s (150 poise) at 25°C.

**44.** A printable composition according to any one of claims 33 to 43, wherein the printable composition shows shear-thinning of at least about 0.1 log viscosity per tenfold increase of shear rate in the range of shear rates about 10 to 100 reciprocal seconds and has a viscosity at 200 reciprocal seconds in the range 0.1 to 3.5 Pa.s (1 to 35 poise) at 25°C.

**45.** A printable composition according to any one of claims 33 to 44, wherein said chemically or electrochemically active component is water-soluble and is selected from enzymes, antigens, antibodies, electron transfer agents able to react with enzymes, and pH buffer materials.

**46.** A printable composition according to any one of claims 33 to 44, wherein said chemically or electrochemically active material is an ion-selective ionophore material.

**47.** A printable composition according to any one of claims 33 to 46, wherein the chemically or electrochemically active substance is dissolved in a solvent for the substance, and wherein the polymeric binder is dissolved or dispersed in said solvent, and comprising from 1 to 15% by weight of a dispersed finely-divided inert particulate material.

**48.** A printable composition according to any one of claims 33 to 46, wherein the polymeric binder is dissolved or dispersed in a solvent, the solvent also having dispersed therein from 5 to 60% by weight of the chemically or electrochemically active substance in finely-divided solid particulate form having a particle size less than 20 $\mu$m.

**Revendications**

**1.** Dispositif d'essai pour effectuer un test microchimique sur un liquide de réaction, qui comprend:

(a) un récipient pour recevoir le liquide de réaction, ce récipient comportant une surface qui, en service, vient en contact avec ledit liquide de réaction ; et

(b) une matière contenant un composant chimiquement ou électrochimiquement actif, déposée suivant un motif défini sur ladite surface du récipient, cette matière étant présente en une quantité comprise entre 1 et 40 mg/cm² et ayant été déposée sur ladite surface par l'impression d'une composition comprenant des particules solides d'une granulométrie de 0,5 à 50 $\mu$m

conjointement avec un liant polymère.

**2.** Dispositif d'essai selon la revendication 1, dans lequel lesdites particules comprennent des particules inertes et sont dispersées dans une matrice comprenant le composant actif et le liant polymère.

**3.** Dispositif d'essai selon la revendication 2, dans lequel lesdites particules inertes comprennent PTFE ou du gel de silice.

**4.** Dispositif d'essai selon la revendication 2 ou 3, dans lequel ledit liant polymère comprend un dérivé cellulosique hydrosoluble.

**5.** Dispositif d'essai selon l'une quelconque des revendications précédentes, dans lequel lesdites particules comprennent le composant actif sous une forme solide finement divisée et sont dispersées dans le liant polymère.

**6.** Dispositif d'essai selon la revendication 5, dans lequel le liant polymère comprend un dérivé cellulosique hydrosoluble.

**7.** Dispositif d'essai selon l'une quelconque des revendications précédentes, dans lequel les particules ont une granulométrie de 1 à 20 $\mu$m.

**8.** Dispositif d'essai selon la revendication 7, dans lequel les particules ont une granulométrie de 3 à 15 $\mu$m.

**9.** Dispositif d'essai selon l'une quelconque des revendications précédentes, dans lequel les particules constituent de 1 à 60% en poids de la composition imprimable.

**10.** Dispositif d'essai selon la revendication 9, dans lequel les particules constituent de 5 à 15% en poids de la composition imprimable.

**11.** Dispositif d'essai selon l'une quelconque des revendications précédentes, dans lequel la matière est présente à raison de 1 à 10 mg/cm².

**12.** Dispositif d'essai selon l'une quelconque des revendications précédentes, dans lequel la composition imprimable présente une viscosité comprise entre 0,1 et 25,0 Pa.s (1 à 250 poises) à 25°C.

**13.** Dispositif d'essai selon la revendicaton 12, dans lequel la viscosité de la composition imprimable est d'au moins 15,0 Pa.s environ (150 poises) à 25°C.

**14.** Dispositif d'essai selon l'une quelconque des revendications précédentes, dans lequel la composition imprimable présente une amincissement par cisaillement d'au moins 0,1 log de viscosité environ pour une augmentation décuple du taux de cisaillement dans l'intervalle de taux de cisaillement d'environ 10 à 100 s$^{-1}$ et sa viscosité à 200 s$^{-1}$ est comprise entre 0,1 et 3,5 Pa.s (1 à 35 poises) à 25°C.

**15.** Dispositif d'essai selon l'une quelconque des revendications précédentes, dans lequel ledit composant chimiquement ou électrochimiquement actif est hydrosoluble et est choisi parmi les enzymes, les antigènes, les anticorps, les agents de transfert d'électrons capables de réagir avec les enzymes et des tampons de pH.

**16.** Dispositif d'essai selon l'une quelconque des revendications 1 à 14, dans lequel ladite matière chimiquement ou électrochimiquement active est un ionophore sélectif aux ions.

**17.** Procédé de fabrication d'un dispositif d'essai selon la revendication 1, qui consiste :
(a) à fournir une composition imprimable contenant un composant chimiquement ou électrochimiquement actif et comprenant des particules solides d'une granulométrie comprise entre 0,5 à 50 $\mu$m conjointement avec un liant polymère ;
(b) à imprimer ladite composition imprimable? pour former un motif défini de matière sur une surface du substrat devant constituer une surface interne d'un récipient pour recevoir un liquide de réaction, ladite matière étant présente en une quantité comprise entre 1 et 40 mg/cm$^2$ ; et
(c) à obliger ou à permettre à ladite matière imprimée de sécher et de se solidifier.

**18.** Procédé selon la revendication 17, dans lequel lesdites particules sont des particules inertes et sont dispersées dans une matrice comprenant le composant actif et le liant polymère.

**19.** Procédé selon la revendicatin 18, dans lequel lesdites particules inertes comprennent PTFE ou du gel de silice.

**20.** Procédé selon la revendication 18 ou 19, ans lequel ledit liant polymère comprend un dérivé cellulosique hydrosoluble.

**21.** Procédé selon la revendication 17, dans lequel lesdites particules comprennent le composant actif sous une forme solide finement divisée et sont dispersées dans le liant polymère.

**22.** Procédé selon l'une quelconque des revendications 17 à 21, dans lequel le liant polymère comprend un dérivé cellulosique hydrosoluble.

**23.** Procédé selon l'une quelconque des revendications 17 à 22, dans lequel la granulométrie des particules est comprise entre 1 et 20 $\mu$m.

**24.** Procédé selon la revendication 23, dans lequel la granulométrie des particules est comprise entre 3 et 15 $\mu$m.

**25.** Procédé selon l'une quelconque des revendications 17 à 24, dans lequel les particules constituent de 1 à 60% en poids de la composition imprimable.

**26.** Procédé selon l'une quelconque des revendications 17 à 24, dans lequel les particules représentent de 5 à 15% en poids de la composition imprimable.

**27.** Procédé selon l'une quelconque des revendications 17 à 26, dans lequel la matière est présente à raison de 1 à 10 mg/cm$^2$.

**28.** Procédé selon l'une quelconque des revendications 17 à 27, dans lequel la viscosité de la composition imprimable est comprise entre 0,1 et 25,0 Pa.s (1 à 250 poises) à 25°C.

**29.** Procédé selon la revendication 28, dans lequel la viscosité de la composition imprimable est d'au moins 15,0 Pa.s (150 poises) à 25°C.

**30.** Procédé selon l'une quelconque des revendications 17 à 29, dans lequel la composition imprimable présente un amincissement par cisaillement d'au moins 0,1 log de viscosité environ, pour une augmentation décuple du taux de cisaillement, ce taux étant compris entre environ 10 et 100 s$^{-1}$ et la viscosité à 200 s$^{-1}$ est comprise entre 0,1 et 3,5 Pa.s (1 à 35 poises) à 25°C.

**31.** Procédé selon l'une quelconque des revendications 17 à 30, dans lequel ledit composant chimiquement ou électrochimiquement actif est hydrosoluble et est choisi parmi les enzymes, les antigènes, les anticorps, les agents de transfert d'électrons capables de réagir avec les enzymes et les tampons de pH.

**32.** Procédé selon l'une quelconque des revendications 17 à 30, dans lequel ladite matière chimiquement ou électrochimiquement active est un ionophore sélectif aux ions.

33. Composition imprimable destinée à imprimer un motif défini de matière sur une surface d'un substrat qui doit former une surface interne d'un récipient pour un dispositif d'essai selon la revendication 1, qui comprend :

(a) une substance chimiquement ou électrochimiquement active ;

(b) des particules solides d'une granulométrie de 0,5 à 50 $\mu$m ; et

(c) un liant polymère.

34. Composition imprimable selon la revendication 33, dans laquelle lesdites particules comprennent des particules inertes et sont dispersées dans une matrice comportant le composant actif et le liant polymère.

35. Composition imprimable selon la revendication 34, dans laquelle lesdites particules inertes comprennent PTFE ou du gel de silice.

36. Composition imprimable selon la revendication 33, dans laquelle lesdites particules constituent le composant actif, sous une forme solide finement divisée et sont dispersées dans le liant polymère.

37. Composition imprimable selon l'une quelconque des revendications 33 à 36, dans laquelle le liant polymère comprend un dérivé cellulosique hydrosoluble.

38. Composition imprimable selon l'une quelconque des revendications 33 à 37, dans laquelle les particules ont une granulométrie de 1 à 20 $\mu$m.

39. Composition imprimable selon la revendication 38, dans laquelle la granulométrie des particules est de 3 à 15 $\mu$m.

40. Composition imprimable selon l'une quelconque des revendications 33 à 39, dans laquelle les particules constituent de 1 à 60% en poids de la composition imprimable.

41. Composition imprimable selon la revendication 40, dans laquelle les particules constituent de 5 à 15% en poids de la composition imprimable.

42. Composition imprimable selon l'une quelconque des revendications 33 à 41, dans laquelle la viscosité de la composition imprimable est comprise entre 0,1 et 25,0 Pa.s (1 et 250 poises) à 25°C.

43. Composition imprimable selon la revendication 42, dans laquelle la viscosité de la composition est d'au moins 15,0 Pa.s (150 poises) à 25°C.

44. Composition imprimable selon l'une quelconque des revendications 33 à 43, dans laquelle l'amincissement par cisaillement est d'au moins 0,1 log de viscosité par augmentation décuple du taux de cisaillement, ce taux étant compris entre 10 et 100 $s^{-1}$ et sa viscosité à 200 $s^{-1}$ est comprise entre 0,1 et 3,5 Pa.s (1 à 35 poises) à 25°C.

45. Composition imprimable selon l'une quelconque des revendications 33 à 44, dans laquelle ledit composant chimiquement ou électrochimiquement actif est hydrosoluble et est choisi parmi les enzymes, les antigènes, les anticorps, les agents de transfert d'électrons capables de réagir avec les enzymes et des tampons de pH.

46. Composition imprimable selon l'une quelconque des revendications 33 à 44, dans laquelle ladite matière chimiquement ou électrochimiquement active est un ionophore sélectif aux ions.

47. Composition imprimable selon l'une quelconque des revendications 33 à 46, dans laquelle la substance chimiquement ou électrochimiquement active est dissoute dans un solvant pour cette substance et, dans laquelle le liant polymère est dissous ou dispersé dans ledit solvant, et comprenant de 1 à 15% en poids d'une matière particulaire inerte finement divisée en dispersion.

48. Composition imprimable selon l'une quelconque des revendications 33 à 46, dans laquelle le liant polymère est dissous ou dispersé dans un solvant, ce solvant contenant également en dispersion de 5 à 60% en poids de la substance chimiquement ou électrochimiquement active sous une forme solide particulaire finement divisée d'une granulométrie inférieure à 20 $\mu$m.

**Patentansprüche**

1. Testvorrichtung zur Durchführung eines mikrochemischen Tests auf eine Reaktionsflüssigkeit, umfassend:

a) einen Behälter zum Sammeln der Reaktionsflüssigkeit, wobei der Behälter eine Oberfläche besitzt, die mit der sich in Gebrauch befindlichen Reaktionsflüssigkeit in Kontakt steht, und

b) Material, einschließlich einer chemisch oder elektrochemisch aktiven Komponente, die in einem definierten Muster auf der Oberfläche des Behälters abgeschieden worden ist, wobei das Material in einer Menge innerhalb des Bereiches von 1 bis 40 mg/cm² vorliegt, und wobei das Material durch Drucken einer Zusammensetzung, die feste Teilchen einer Teilchengröße im Bereich von 0,5 bis 50 Mikrometern, zusammen mit einem polymeren Bindemittel enthält, auf der Oberfläche abgeschieden worden ist.

2. Testvorrichtung nach Anspruch 1, wobei die Teilchen inerte Teilchen umfassen und in eine Matrix aus der aktiven Komponente und dem polymeren Bindemittel dispergiert worden sind.

3. Testvorrichtung nach Anspruch 2, wobei die inerten Teilchen PTFE oder Kieselgel umfassen.

4. Testvorrichtung nach Anspruch 2 oder 3, wobei das polymere Bindemittel ein wasserlösliches Cellulosederivat umfaßt.

5. Testvorrichtung nach irgendeinem der vorangegangenen Ansprüche, wobei die Teilchen die aktive Komponente in fester feinverteilter Form umfassen und wobei die Teilchen in dem polymeren Bindemittel dispergiert sind.

6. Testvorrichtung nach Anspruch 5, wobei das polymere Bindemittel ein wasserlösliches Cellulosederivat umfaßt.

7. Testvorrichtung nach irgendeinem der vorangegangenen Ansprüche, wobei die Teilchen eine Teilchengröße im Bereich von 1 bis 20 Mikrometern besitzen.

8. Testvorrichtung nach Anspruch 7, wobei die Teilchen eine Teilchengröße im Bereich von 3 bis 15 Mikrometern besitzen.

9. Testvorrichtung nach irgendeinem der vorangegangenen Ansprüche, wobei die Teilchen in einer Menge von 1 bis 60 Gew.-% der druckfähigen Zusammensetzung eingesetzt werden.

10. Testvorrichtung nach Anspruch 9, wobei die Teilchen in einer Menge von 5 bis 15 Gew.-% der druckfähigen Zusammensetzung eingesetzt werden.

11. Testvorrichtung nach irgendeinem der vorangegangen Ansprüche, wobei das Material in einer Menge im Bereich von 1 bis 10 mg/cm² vorliegt.

12. Testvorrichtung nach irgendeinem der vorangegangenen Ansprüche, wobei die druckfähige Zusammensetzung eine Viskosität im Bereich von 0,1 bis 25,0 Pa.s (1 bis 250 Poise) bei 25°C besitzt.

13. Testvorrichtung nach Anspruch 12, wobei die druckfähige Zusammensetzung eine Viskosität von mindestens ungefähr 15,0 Pa.s (150 Poise) bei 25°C besitzt.

14. Testvorrichtung nach irgendeinem der vorangegangen Ansprüche, wobei die druckfähige Zusammensetzung eine Scherentzähung von mindestens ungefähr 0,1 log Viskosität pro zehnfacher Erhöhung der Scherrate im Bereich von Scherraten von etwa 10 bis 100 reziproken Sekunden zeigt und wobei die Zusammensetzung eine Viskosität im Bereich von 0,1 bis 3,5 Pa.s (1 bis 35 Poise) bei 200 reziproken Sekunden bei 25°C aufweist.

15. Testvorrichtung nach irgendeinem der vorangegangen Ansprüche, wobei die chemisch oder elektrochemisch aktive Komponente wasserlöslich ist und unter Enzymen, Antigenen, Antikörpern, Elektronenübertragungsmitteln, die geeignet sind, mit den Enzymen zu reagieren und pH-Puffermaterialien, ausgewählt ist.

16. Testvorrichtung nach irgendeinem der Ansprüche 1 bis 14, wobei das chemisch oder elektrochemisch aktive Material ein ionenselektives ionophores Material ist.

17. Verfahren zur Herstellung einer Testvorrichtung nach Anspruch 1, umfassend:

a) Bereitstellen einer druckfähigen Zusammensetzung, die eine chemisch oder elektrochemisch aktive Komponente einschließt und die feste Teilchen mit einer Teilchengröße im Bereich von 0,5 bis 50 Mikrometern, zusammen mit einem polymeren Bindemittel umfaßt,

b) Drucken der druckfähigen Zusammensetzung zur Bildung eines definierten Materialmusters auf der Oberfläche eines Substrats, welches eine innere Oberfläche eines Behälters bildet, um eine Reaktionsflüssigkeit zu erhalten, wobei das Material in einer Menge im Bereich von 1 bis 40 mg/cm² vorliegt, und

c) Verursachen oder Gestatten des Trocknens oder Verfestigens des gedruckten Materials.

**18.** Verfahren nach Anspruch 17, wobei die Teilchen inerte Teilchen umfassen und in einer Matrix, die die aktive Komponente und das polymere Bindemittel enthält, dispergiert sind.

**19.** Verfahren nach Anspruch 18, wobei die inerten Teilchen PTFE oder Kieselgel umfassen.

**20.** Verfahren nach Anspruch 18 oder 19, wobei das polymere Bindemittel ein wasserlösliches Cellulosederivat umfaßt.

**21.** Verfahren nach Anspruch 17, wobei die Teilchen die aktive Komponente in fester feinverteilter Form umfassen und in dem polymeren Bindemittel dispergiert sind.

**22.** Verfahren nach irgendeinem der vorangegangenen Ansprüche 17 bis 21, wobei das polymere Bindemittel ein wasserlösliches Cellulosederivat umfaßt.

**23.** Verfahren nach irgendeinem der Ansprüche 17 bis 22, wobei die Teilchen eine Teilchengröße im Bereichvon 1 bis 20 Mikrometern besitzen.

**24.** Verfahren nach Anspruch 23, wobei die Teilchen eine Teilchengröße im Bereich von 3 bis 15 Mikrometern besitzen.

**25.** Verfahren nach irgendeinem der Ansprüche 17 bis 24, wobei die Teilchen 1 bis 60 Gew.-% der druckfähigen Zusammensetzung ausmachen.

**26.** Verfahren nach irgendeinem der Ansprüche 17 bis 24, wobei die Teilchen 5 bis 15 Gew.-% der druckfähigen Zusammensetzung ausmachen.

**27.** Verfahren nach irgendeinem der Ansprüche 17 bis 26, wobei das Material in einer Menge im Bereich von 1 bis 10 $mg/cm^2$ vorliegt.

**28.** Verfahren nach irgendeinem der Ansprüche 17 bis 27, wobei die druckfähige Zusammensetzung eine Viskosität im Bereich von 0,1 bis 25,0 Pa.s (1 bis 250 Poise) bei 25°C besitzt.

**29.** Verfahren nach Anspruch 28, wobei die druckfähige Zusammensetzung eine Viskosität von mindestens ungefähr 15,0 Pa.s (150 Poise) bei 25°C besitzt.

**30.** Verfahren nach irgendeinem der vorangegangenen Ansprüche 17 bis 29, wobei die druckfähige Zusammensetzung eine Scherentzähung von mindestens ungefähr 0,1 log Viskosität pro zehnfacher Erhöhung der Scherrate im Bereich von Scherraten von ungefähr 10 bis 100 reziproken Sekunden aufweist und wobei die Zusammensetzung eine Viskosität im Bereich von 0,1 bis 3,5 Pa.s (1 bis 35 Poise) bei 200 reziproken Sekunden und 25°C besitzt.

**31.** Verfahren nach einem der vorangegangenen Ansprüche 17 bis 30, wobei die chemisch oder elektrochemisch aktive Komponente wasserlöslich ist und unter Enzymen, Antigenen, Antikörpern, Elektronenübertragungsmitteln, die mit Enzymen reagieren können und pH-Puffermaterialien, ausgewählt wird.

**32.** Verfahren nach einem der Ansprüche 17 bis 30, wobei das chemisch oder elektrochemisch aktive Material ein ionenselektives ionophores Material ist.

**33.** Druckfähige Zusammensetzung zum Drucken eines definierten Materialmusters auf einer Substratoberfläche, die eine innere Oberfläche eines Behälters für eine Testvorrichtung nach Anspruch 1 bildet, umfassend:

    a) eine chemisch oder elektrochemisch aktive Substanz,
    b) feste Teilchen mit einer Teilchengröße im Bereich von 0,5 bis 50 Mikrometern, und
    c) ein polymeres Bindemittel.

**34.** Druckfähige Zusammensetzung nach Anspruch 33, wobei die Teilchen inerte Teilchen enthalten und in einer Matrix aus der aktiven Komponente und dem polymeren Bindemittel dispergiert sind.

**35.** Druckfähige Zusammensetzung nach Anspruch 34, wobei die inerten Teilchen PTFE oder Kieselgel umfassen.

**36.** Druckfähige Zusammensetzung nach Anspruch 33, wobei die Teilchen die aktive Komponente in fester feinverteilter Form umfassen und in dem polymeren Bindemittel dispergiert sind.

**37.** Druckfähige Zusammensetzung nach irgendeinem der Ansprüche 33 bis 36, wobei das polymere Bindemittel ein wasserlösliches Cellulosederivat umfaßt.

**38.** Druckfähige Zusammensetzung nach irgendeinem der Ansprüche 33 bis 37, wobei die Teilchen eine Teilchengröße im Bereich von 1 bis 20 Mikrometern besitzen.

**39.** Druckfähige Zusammensetzung nach Anspruch 38, wobei die Teilchen eine Teilchengroße im Bereich von 3 bis 15 Mikrometern besitzen.

**40.** Druckfähige Zusammensetzung nach irgendeinem der Ansprüche 33 bis 39, wobei die Teilchen 1 bis 60 Gew.-% der druckfähigen Zusammensetzung ausmachen.

**41.** Druckfähige Zusammensetzung nach Anspruch 40, wobei die Teilchen 5 bis 15 Gew.-% der druckfähigen Zusammensetzung ausmachen.

**42.** Druckfähige Zusammensetzung nach irgendeinem der Ansprüche 33 bis 41, wobei die druckfähige Zusammensetzung eine Viskosität im Bereich von 0,1 bis 25,0 Pa.s (1 bis 250 Poise) bei 25°C besitzt.

**43.** Druckfähige Zusammensetzung nach Anspruch 42, wobei die druckfähige Zusammensetzung eine Viskosität von mindestens ungefähr 15,0 Pa.s (150 Poise) bei 25°C besitzt.

**44.** Druckfähige Zusammensetzung nach irgendeinem der Ansprüche 33 bis 43, wobei die druckfähige Zusammensetzung eine Scherentzähung von mindestens ungefähr 0,1 log Viskosität pro zehnfacher Erhöhung der Scherrate im Bereich von Scherraten von 10 bis 100 reziproken Sekunden aufweist und wobei die Zusammensetzung eine Viskosität im Bereich von 0,1 bis 3,5 Pa.s (1 bis 35 Poise) bei 200 reziproken Sekunden und 25°C besitzt.

**45.** Druckfähige Zusammensetzung nach irgendeinem der Ansprüche 33 bis 44, wobei die chemisch oder elektrochemisch aktive Komponente wasserlöslich ist und unter Enzymen, Antigenen, Antikörpern, Elektronenübertragungsmitteln, die geeignet sind, mit Enzymen zu reagieren, und pH-Puffermaterialien ausgewählt ist.

**46.** Druckfähige Zusammensetzung nach irgendeinem der Ansprüche 33 bis 44, wobei das chemisch oder elektrochemisch aktive Material ein ionenselektives ionophores Material ist.

**47.** Druckfähige Zusammensetzung nach irgendeinem der Ansprüche 33 bis 46, wobei die chemisch oder elektrochemisch aktive Substanz in einem Lösungsmittel für die Substanz gelöst ist das polymere Bindemittel in dem Lösungsmittel gelöst oder dispergiert ist, und die Substanz 1 bis 15 Gew.-% eines dispergierten feinverteilten inerten Teilchenmaterials enthält.

**48.** Druckfähige Zusammensetzung nach irgendeinem der Ansprüche 33 bis 46, wobei das polymere Bindemittel in einem Lösungsmittel gelöst oder dispergiert ist und wobei im Lösungsmittel auch 5 bis 60 Gew.-% der chemisch oder elektrochemisch aktiven Substanz in feinverteilter fester Partikelform mit einer Teilchengröße kleiner als 20 Mikrometer, dispergiert sind.

# Fig.1.